# EUROPÄISCHE PATENTSCHRIFT

(11) **EP 2 405 893 B1**
(45) Veröffentlichungstag und Bekanntmachung des Hinweises auf die Patenterteilung: **22.04.2015**
(21) Anmeldenummer: 10707228.2
(22) Anmeldetag: 05.03.2010
(51) Int. Cl.: A61K 38/15, A61K 9/10, A61K 31/53, A61K 9/14, A61K 38/12

(54) **Zubereitung auf Ölbasis enthaltend anti-protozoische Triazine und anthelmintische Cyclodepsipeptide**
Oil-based composition comprising anti-protozoal trazines and anthelminthic cyclodepsipeptides
Compositions à base d'huile comprenant des triazines anti-protozoiques et des cyclodepsipeptides anthelminthiques

(30) Priorität: 10.03.2009 DE 102009012423
(43) Veröffentlichungstag der Anmeldung: 18.01.2012
(73) Patentinhaber: Bayer Intellectual Property GmbH, 40789 Monheim (DE)
(72) Erfinder: KANIKANTI, Venkata-Rangarao, 51381 Leverkusen (DE); HARDER, Achim, 51109 Köln (DE); BACH, Thomas, 42349 Wuppertal (DE)
(74) Vertreter: BIP Patents
(86) Internationale Anmeldenummer: PCT/EP2010/001396
(87) Internationale Veröffentlichungsnummer: WO 2010/102762

(56) Entgegenhaltungen:
- EP-A1- 0 626 376
- US-A- 3 966 725
- EL-BANNA H A ET AL: "Comparative pharmacokinetics of ivermectin alone and a novel formulation of ivermectin and rafoxanide in calves and sheep" PARASITOLOGY RESEARCH ; FOUNDED AS ZEITSCHRIFT FÜR PARASITENKUNDE, SPRINGER, BERLIN, DE, Bd. 102, Nr. 6, 23. Februar 2008 (2008-02-23), Seiten 1337-1342, XP019630895 ISSN: 1432-1955
- PODDAR AVIJIT ET AL: "In vivo efficacy of calceolarioside a against experimental visceral leishmaniasis" PLANTA MEDICA, Bd. 74, Nr. 5, April 2008 (2008-04), Seiten 503-508, XP002579877 ISSN: 0032-0943
- BARUTZKI D ET AL: "Endoparasites in dogs and cats in Germany 1999-2002" PARASITOLOGY RESEARCH, SPRINGER VERLAG, BERLIN, DE LNKD- DOI:10.1007/S00436-003-0922-6, Bd. 90, Nr. suppl. 3, 1. Juli 2003 (2003-07-01), Seiten S148-S150, XP009132650 ISSN: 0932-0113
- GEARY T G ET AL: "DEVELOPMENT OF ANTIPARASITIC DRUGS IN THE 21ST CENTURY" VETERINARY PARASITOLOGY, ELSEVIER SCIENCE, AMSTERDAM, NL LNKD- DOI:10.1016/S0304-4017(03)00205-X, Bd. 115, Nr. 2, 1. Januar 2003 (2003-01-01), Seiten 167-184, XP008037833 ISSN: 0304-4017

## Beschreibung

Die vorliegende Erfindung betrifft eine Zubereitung auf Ölbasis enthaltend ein gegen parasitische Protozoen wirksames Triazin und ein anthelminthisches Cyclodepsipeptid, die sich insbesondere zur oralen Anwendung der Wirkstoffkombination bei Tieren eignet.

Triazine, inbesondere Toltrazuril und Ponzazuril, sowie ihre Wirkung gegen parasitische Protozoen, wie z. B. Coccidien, sind aus einer Reihe von Veröffentlichungen bekannt, siehe u. a. DE-OS 27 18 799, DE-OS 24 137 22, EP-A 116 175, EP-A 1 246 624, EP-A 1 140 102, EP-A 1 311 491 und WO 2008/145281. Aus WO 99/62519 sind halbfeste wässrige Zubereitungen von Toltrazuril-Sulfon (Ponazuril) bekannt.

Cyclische Depsipeptide und ihre endoparasitizide Wirkung sind bekannt: Enniatine und andere 18-gliedrige cyclische Depsipeptide (EP-A 644 883, EP-A 658 551, EP-A 669 343, WO 95/27498); 24-gliedrige cyclische Depsipeptide (EP-A 626 376, EP-A 626 375, EP 787 141, EP-A 903 347, EP-A 973 756, WO 98/55469, WO 99/47506, WO 00/14079, WO 98/37088, WO 99/67281), cyclische Depsipeptide mit 12 Ringatomen (EP-A 664 297). Cyclische Oktadepsipeptide wie PF1022 und Emodepsid und ihre Wirkung gegen Endoparasiten (z.B. gegen Darmnematoden und Gewebsnematoden) sind ebenfalls bereits bekannt, siehe z.B. EP-A 382 173, EP-A 634 408. Weiterhin seien im Zusammenhang mit Depsipeptiden, insbesondere Emodepsid genannt: EP 662 326, EP-A 1 259 250 und WO05/055973.

Es bestand daher ein Bedürfnis an pharmazeutischen Zubereitungen, die insbesondere für die orale Anwendung geeignet sind und gegen parasitische Protozoen wirksame Triazine sowie anthelmintische Depsipeptide enthalten (siehe z. B. Barutzki D; Schaper R "Endoparasites in dogs and cats in Germany 1999-2002". Parasitol. Res. 2003 Jul; 90 Suppl. 3:S. 148-50. Epub 2003, Aug. 19). Die Triazine müssen systemisch zur Verfügung stehen, d.h. vor allem bei oraler Verabreichung müssen sie aus dem Magen-Darm-Trakt in den Blutkreislauf übergehen, um dort gegen die parasitischen Protozoen zu wirken. Im Gegensatz dazu sollten die anthelmintischen Depsipeptide ihre Wirkung im Darm entfalten, da die Würmer dort lokal bekämpft werden sollen. Ein Übergang in den Blutkreislauf ist nicht erforderlich und eher unerwünscht, um etwaige Nebenwirkungen zu vermeiden.

Die Aufgabe bestand also darin, eine Formulierung zu finden, die das Depsipeptid lokal im Darm und das Triazin im Blutkreislauf verfügbar macht. Wichtig ist dabei, dass die beiden Wirkstoffe jeweils in ausreichender Menge am richtigen Wirkort zur Verfügung stehen. Das Depsipeptid sollte nur in solchen Mengen in den Blutkreislauf gelangen, die bezüglich Nebenwirkungen beim behandelten Tier unkritisch sind.

Die Erfindung betrifft: Zubereitungen enthaltend ein gegen parasitische Protozoen wirksames Triazin mit einer Korngröße d(90) ≤ 15 µm und ein anthelmintisches Cyclodepsipeptid in einer Ölbasis.

Gegen parasitische Protozoen wirksame Triazine sind insbesondere solche der Formeln (I) oder (II) 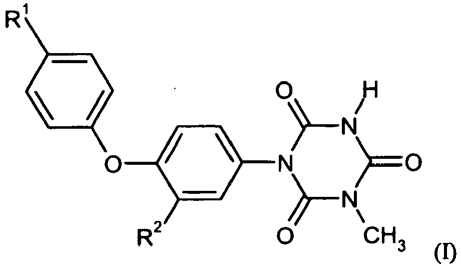 oder 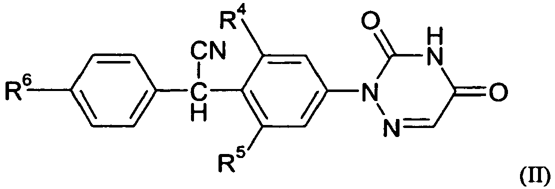 worin
- R¹: für R³-SO₂- oder R³-S- steht,
- R²: für Alkyl, Alkoxy, Halogen oder SO₂N(CH₃)₂ steht und
- R³: für Halogenalkyl steht
- R⁴ und R⁵: unabhängig voneinander für Wasserstoff oder Cl stehen und
- R⁶: für Fluor oder Chlor steht, sowie ihre physiologisch verträglichen Salze

Die Triazine sind als Wirkstoffe vor allem gegen Coccidien-Infektionen per se gut bekannt, genannt seien die Triazintrione wie z.B. Toltrazuril und Ponazuril sowie die Triazindione wie z.B. Clazuril, Diclazuril und Letrazuril.

Die Triazindione werden durch Formel (II) wiedergegeben:
Clazuril (R⁴ = Cl, R⁵ = H, R⁶ = Cl in Formel (II)
Letrazuril (R⁴ = Cl, R⁵ = Cl, R⁶ = F in Formel (II)) und
Diclazuril (R⁴ = Cl, R⁵ = Cl, R⁶ = Cl in Formel (II)).

Von diesen 1,2,4-Triazindionen ist Diclazuril am meisten bevorzugt.

Erfindungsgemäß besonders bevorzugt sind die Triazintrione der Formel (I) als Wirkstoffe:
- R²: steht bevorzugt für Alkyl oder Alkoxy mit jeweils bis zu 4 Kohlenstoffatomen, besonders bevorzugt für Methyl, Ethyl, n-Propyl, i-Propyl.
- R³: steht bevorzugt für Perfluoralkyl mit 1 bis 3 Kohlenstoffatomen, besonders bevorzugt für Trifluormethyl oder Pentafluorethyl.

Die bevorzugten Triazintrione werden durch die Formel (I) wiedergegeben:
Toltrazuril (R¹ = R³-S-, R² = CH₃, R³ = CF₃)
Ponazuril (R¹ = R³-SO₂-, R² = CH₃, R³ = CF₃)

Depsipeptide sind den Peptiden ähnlich und unterscheiden sich von diesen darin, dass ein oder mehrere α-Aminosäurebausteine durch α-Hydroxycarbonsäurebausteine ersetzt sind. Erfindungsgemäß bevorzugt eingesetzt werden cyclische Depsipeptide insbesondere mit 24 Ringatomen (Cyclooctadepsipeptide).

Unter den cyclischen Depsipeptiden mit 24 Ringatomen sei die aus EP-OS 382 173 bekannte Verbindung PF 1022 der folgenden Formel (IIIa) genannt:

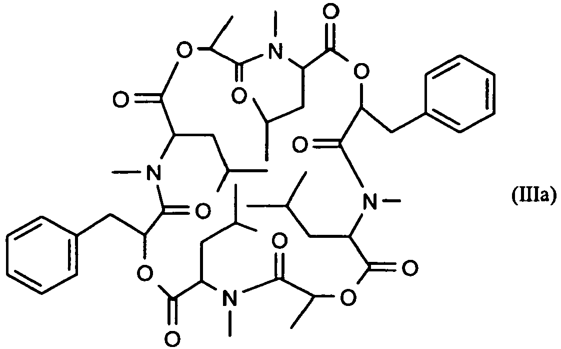

Außerdem seien als Depsipeptide die aus der PCT-Anmeldung WO 93/19053 bekannten Verbindungen genannt.

Insbesondere seien aus WO 93/19053 die Verbindungen der folgenden Formel (IIIb) genannt: 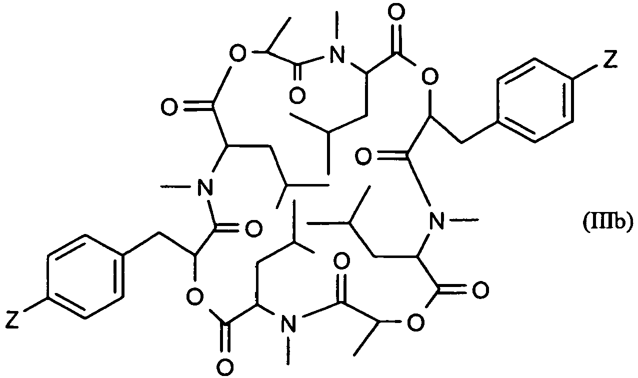 in welcher
- Z: für N-Morpholinyl, Amino, Mono- oder Dimethylamino steht.

Außerdem seien Verbindungen der folgenden Formel (IIIc) genannt: 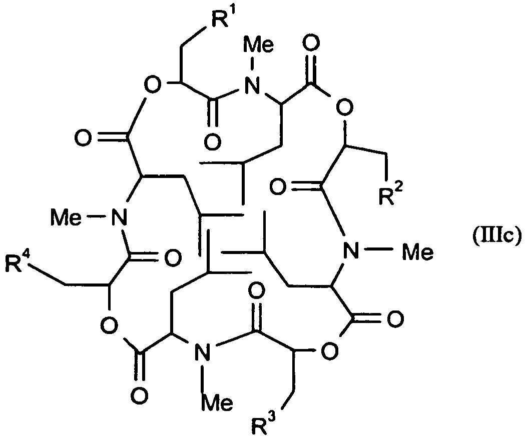 in welcher
R¹, ^{R}2, R³, R⁴ unabhängig voneinander für Wasserstoff, C₁-C₁₀-Alkyl oder Aryl, insbesondere Phenyl stehen, die gegebenenfalls substituiert sind durch Hydroxy, C₁-C₁₀-Alkoxy oder Halogen.

Zu den cyclischen Depsipeptiden mit 24 Ringatomen zählen auch Verbindungen der allgemeinen Formel (IIId) 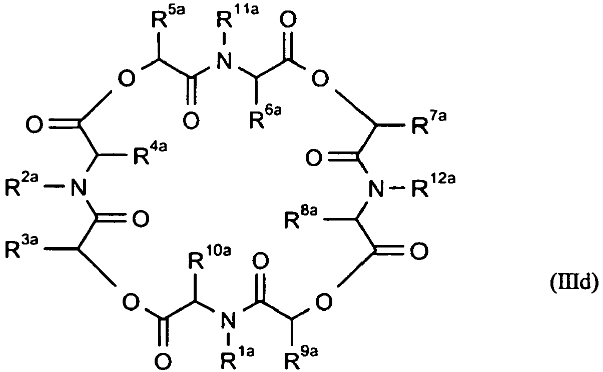 in welcher
R^{1a}, R^{2a}, R^{11a} und R^{12a} unabhängig voneinander für C₁₋₈-Alkyl, C₁₋₈-Halogenalkyl, C₃₋₆-Cycloalkyl, Aralkyl, Aryl stehen,
R^{3a}, R^{5a}, R^{7a}, R^{9a} unabhängig voneinander für Wasserstoff oder geradkettiges oder verzweigtes C₁₋₈-Alkyl steht, das gegebenenfalls durch Hydroxy, C₁₋₄-Alkoxy, Carboxy, 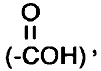 Carboxamid, 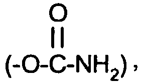 Imidazolyl, Indolyl, Guanidino, -SH oder C₁₋₄-Alkylthio substituiert sein kann und ferner für Aryl oder Aralkyl die durch Halogen, Hydroxy, C₁₋₄-Alkyl, C₁₋₄-Alkoxy substituiert sein können, steht,
R^{4a}, R^{6a}, R^{8a}, R^{10a} unabhängig voneinander für Wasserstoff, geradkettiges C₁₋₅-Alkyl, C₂₋₆-Alkenyl, C₃₋₇-Cycloalkyl, die gegebenenfalls durch Hydroxy, C₁₋₄-Alkoxy, Carboxy, Carboxamid, Imidazolyl, Indolyl, Guanidino, SH oder C₁₋₄-Alkylthio substituiert sein können, sowie für Aryl oder Aralkyl die durch Halogen, Hydroxy, C₁₋₄-Alkyl, C₁₋₄-Alkoxy substituiert sein können, stehen sowie deren optische Isomere und Racemate.

Bevorzugt werden Verbindungen der Formel (IIId) eingesetzt, in welcher
R^{1a}, R^{2a} , R^{11a} und R^{12a} unabhängig voneinander für Methyl, Ethyl, Propyl, Isopropyl, n-, s-, t-Butyl oder Phenyl, das gegebenenfalls substituiert ist durch Halogen, C₁₋₄-Alkyl, OH, C₁₋₄-Alkoxy, sowie für Benzyl oder Phenylethyl stehen, die gegebenenfalls durch die bei Phenyl angegebenen Reste substituiert sein können;
R^{3a} bis R^{10a} die oben angegebene Bedeutung haben.

Besonders bevorzugt sind Verbindungen der Formel (IIId), in welcher
R^{1a}, R^{2a}, R^{11a} und R^{12a} unabhängig voneinander für Methyl, Ethyl, Propyl, Isopropyl oder n-, s-, t-Butyl stehen,
R^{3a}, R^{5a}, R^{7a}, R^{9a} für Wasserstoff, geradkettiges oder verzweigtes C₁₋₈-Alkyl, insbesondere Methyl, Ethyl, Propyl, i-Propyl, n-, s-, t-Butyl, die gegebenenfalls durch C₁₋₄-Alkoxy, insbesondere Methoxy, Ethoxy, Imidazolyl, Indolyl oder C₁₋₄-Alkylthio, insbesondere Methylthio, Ethylthio substituiert sein können, ferner für Phenyl, Benzyl oder Phenethyl stehen, die gegebenenfalls durch Halogen insbesondere Chlor substituiert sein können.

R^{4a}, R^{6a}, R^{8a}, R^{10a} unabhängig voneinander für Wasserstoff, Methyl, Ethyl, n-Propyl, n-Butyl, Vinyl, Cyclohexyl, die gegebenenfalls durch Methoxy, Ethoxy, Imidazolyl, Indolyl, Methylthio, Ethylthio substituiert sein können, sowie für Isopropyl, s-Butyl ferner für gegebenenfalls halogensubstituiertes Phenyl, Benzyl oder Phenylethyl stehen.

Die o. g. Cyclooctadepsipeptide sind bekannt und mit bekannten Verfahren herstellbar. So können sie z. B. nach den in EP-A-382 173, DE-A 4 317 432, DE-A 4 317 457, DE-A 4 317 458, EP-A-634 408, EP-A-718 293, EP-A-872 481, EP-A-685 469, EP-A-626 375, EP-A-664 297, EP-A-669 343, EP-A-787 141, EP-A-865 498, EP-A-903 347 beschriebenen Verfahren erhalten werden.

Erfindungsgemäß ganz besonders bevorzugte Depsipeptide sind PF 1022 A (siehe Formel (IIIa) und Emodepside (PF 1022-221, Verbindung der Formel (IIIb) worin beide Reste Z für den Morpholinylrest stehen). Der INN Emodepside steht für die Verbindung mit dem systematischen Namen: Cyclo[(*R*)-lactoyl-*N*-methyl-L-leucyl-(*R*)-3-(*p*-morpholinophenyl)lactoyl-*N*-methyl-L-leucyl-(*R*)-lactoyl-*N*-methyl-L-leucyl-(*R*)-3-(*p*-morpholinophenyl)lactoyl-N-methyl-L-leucyl.

Wirkstoffe können je nach Struktur in stereoisomeren Formen oder als Stereoisomerengemische vorliegen, z. B. als Enantiomere oder Racemate bzw. Diastereomere oder Diastereomerengemische. Sowohl die Stereoisomerengemische als auch die reinen Stereoisomeren können erfindungsgemäß verwendet werden, z. B. können die Enantiomeren oder Diastereomeren oder ihre jeweiligen Mischungen eingesetzt werden.

Sofern die Wirkstoffe in tautomeren Formen vorkommen können, umfasst die vorliegende Erfindung auch den Einsatz der tautomeren Formen.

Weiterhin können gegebenenfalls verwendet werden: Salze der Wirkstoffe mit pharmazeutisch annehmbaren Säuren oder Basen und auch Solvate, insbesondere Hydrate, der Wirkstoffe oder ihrer Salze.

Die Wirkstoffe können gegebenenfalls auch in Form ihrer Salze, Solvate und Solvate der Salze eingesetzt werden

Als Salze sind im Rahmen der vorliegenden Erfindung physiologisch unbedenkliche Salze der Wirkstoffe bevorzugt.

Physiologisch unbedenkliche Salze der Wirkstoffe umfassen je nach Struktur des Wirkstoffs Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der Wirkstoffe umfassen gegebenenfalls auch Salze üblicher Basen, wie beispielhaft und vorzugsweise Alkalimetallsalze (z.B. Natrium- und Kaliumsalze), Erdalkalisalze (z. B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin, *N*-Methylpiperidin und Cholin.

Als Solvate werden im Rahmen der Erfindung solche Formen der Wirkstoffe bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem umfasst die vorliegende Erfindung auch Prodrugs der Wirkstoffe. Der Begriff "Prodrugs" umfasst Verbindungen, welche selbst biologisch aktiv oder inaktiv sein können, jedoch während ihrer Verweilzeit im Körper zu dem eigentlichen Wirkstoff umgesetzt werden (beispielsweise metabolisch oder hydrolytisch).

Die erfindungsgemäßen Erzeugnisse können gegebenenfalls weitere Wirkstoffe enhalten. Als bevorzugte Beispiele seien genannt Praziquantel oder makrocyclische Laktone (z.B. Ivermectin, Moxidectin u. a.).

Die Wirkstoffe liegen in den erfindungsgemäßen Zubereitungen in einer Ölbasis vor. Da sie in der Regel in der Ölbasis wenig löslich sind, werden sie üblicherweise darin suspendiert. Als Ölbasis kommen vorzugsweise flüssige pharmazeutisch verträgliche ölige Substanzen in Frage. Vorzugsweise sind dies pharmazeutisch verträgliche ölige Fettsäuretriglyceride, insbesondere solche mit aliphatischen Fettsäuren die 8 bis 20 Kohlenstoffatome enthalten. Besonders bevorzugte Beispiele sind natürliche Pflanzenöle, wie z. B. Sonnenblumenöl, Sojaöl, Rhizinusöl, Sesamöl, Mandelöl, Rapsöl, Weizenkeimöl oder Fischöl oder auch mittelkettige Triglyceride (MTK) wie z. B. Capryl-Caprinsäure-Triglycerid (Miglyol® 812). Weiterhin kommen als Ölbasis die natürlichen Pflanzenöle Aprikosenkemöl, Canolaöl oder modifizierte Pflanzenöle wie "*maleated soybean oil*" (maleiertes Sojaöl) in Frage. Besonders bevorzugt ist Sonnenblumenöl. Die als Ölbasis geeigneten Stoffe können auch als Gemische eingesetzt werden. Die Zubereitungen enthalten die Ölbasis üblicherweise in Mengen von 10 bis 99 Gew.-%, vorzugsweise 50 bis 98 Gew.-%, besonders bevorzugt 80 bis 95 Gew.%.

Die Triazine werden in mikronisierter Form eingesetzt. Für die Mikronisierung können übliche Verfahren verwendet werden, wie z.B. die Perlmahlung die zweckmäßigerweise im vorliegenden Fall gleich im geeigneten öligen Medium durchgeführt werden kann. Dabei weist das dispergierte Triazin eine Partikelgröße (gemessen mit Laserbeugung, Malvem Mastersizer® 2000) von d(90) ≤ 15µm, bevorzugt d(90) ≤ 12µm, besonders bevorzugt d(90) ≤ 10µm, und ganz besonders bevorzugt d(90) kleiner oder gleich 9µm auf. Im Sinne dieser Erfindung wird unter d(90) eine volumenbezogene Partikelgrößenverteilung verstanden, bei der 90% aller Partikel eine Dimension (Durchmesser) kleiner oder gleich diesen Wertes aufweisen. Die hier angegebenen Partikelgrößen wurden mit der Methode der Laserbeugung mit dem Gerät Mastersizer 2000 (Dispergiereinheit Hydro 2000G) der Firma Malvem und dem Auswertemodus der Fraunhoferbeugung bestimmt, da die Brechungindices der Wirkstoffpartikel nicht bekannt sind. Eine geeignete Menge der Probe wird dabei mit 2-3ml eines Dispergiermediums (dünnflüssiges Paraffin) unter Rühren vordispergiert. Die Dispersion wird dann in die Dispergiereinheit des Geräts gegeben und vermessen. Die Auswertesoftware gibt die Partikelgröße als d(90)-Werte usw. aus.

Bevorzugt enthalten die erfindungsgemäßen Zubereitungen weiterhin ein Verdickungsmittel wie z. B. kolloidales Siliziumdioxid oder feste Fettgrundlagen, inbesondere Glycerolester. Bevorzugt handelt es sich um Ester mit C12-C24-Fettsäuren. Als Glycerolester seien genannt Glyceroldiester, wie z. B. Glyceroldibehenat (Compritol 888 ATO); Glyceroltriester, wie z. B. Glycerolester gesättigter C12-C18-Fettsäuren (z. B. Gelucire® 43/01), Glyceroltrilaurat, Glyceroltrimyristat, Glyceroltripalmitat oder Glyceroltristearat (z. B. Dynasan 118), Mischungen aus Glycerolmono-, -di- und -triestern, wie z.B. Glycerolpalmitostearat (Precirol ATO 5). Auch genannt seien Triglyceride auf Basis von Kokosfett, Palmöl und/oder Palmkernöl (wie z. B. die unter der Bezeichnung Witocan® im Handel befindlichen Hartfette). Auch Mono- oder Diglyceride von Zitronen- und/oder Milchsäure sind einsetzbar. Als Beispiel sei genannt: Glycerylstearatcitrat (Imwitor 372P). Weiterhin seien genannt: Glycerylmonoester, wie z. B. Glycerylmonostearat (Imwitor 491); Linoleoylmakrogolglyceride (z. B. Labrafil M2125 CS); Gehärtete Triglyceride auf Basis von Kokos- oder Palmkem-Öl, wie z. B. "*hydrogenated Coco-glycerides*" (z. B. Witocan 42/44); hydriertes Rizinusöl (z. B. Cutina HR PH). Bevorzugt eingesetzt werden Glyceroldiester mit C12-C24 Fettsäuren, als besonders bevorzugtes Beispiel sei Glyceryldibehenat genannt. Das Verdickungsmittel wird üblicherweise in Mengen von 1 bis 15 Gew.-%, bevorzugt 1 bis 10 Gew.-%, besonders bevorzugt 2 bis 8 Gew.-% eingesetzt. Sofern eine flüssige Suspension hergestellt werden soll werden in der Regel höchstens 5 Gew.-% an Verdickungsmittel zugesetzt.

Möglich ist aber auch die Herstellung von Pasten, die in der Regel mehr als 5 Gew.-% Verdickungsmittel enthalten. Unter einer Paste werden hier solche Zubereitungen verstanden, die unter ihrem Eigengewicht nicht fließen (wie dies z. B. von Zahnpasta bekannt ist).

Es wurde gefunden, dass inbesondere bei Verwendung der oben genannten bevorzugten und besonders bevorzugten Verdickungsmittel Zubereitungen mit sehr guter Stabilität gegenüber Sedimentation und Abbau erhalten wurden.

Weiterhin enthalten die erfindungsgemäßen Zubereitungen vorzugsweise ein Antioxidans. Antioxidantien sind z.B. Butylhydroxytoluol, Butylhydroxyanisol, Propylgallat oder Tocopherol oder Kombinationen dieser Antioxidantien. Bevorzugt eingesetzt werden Butylhydroxyanisol (BHA) oder insbesondere Butylhydroxytoluol (BHT) oder Kombinationen dieser Antioxidantien. Diese werden in den für das jeweilige Antioxidans üblichen Mengen eingesetzt. In Regel liegen die Konzentrationen bei 0,01 bis 3 Gew.-%, bevorzugt 0,04 bis 0,5 Gew.-%.

Weiterhin enthalten die Zubereitungen vorzugsweise ein Konservierungsmittel, z. B. para-Hydroxybenzoesäureester (Parabene) wie 4-Hydroxybenzoesäuremethylester, 4-Hydroxybenzoesäureethylester oder 4-Hydroxybenzoesäurepropylester. Bevorzugt wird Sorbinsäure eingesetzt. Die Konservierungsmittel können für eine ausreichende Konservierung einzeln oder in Kombination eingesetzt werden. Sie sind üblicherweise in Konzentrationen von 0,01 bis 1 Gew.-%, bevorzugt 0,02 bis 0,5 Gew.-%, besonders bevorzugt 0,02 bis 0,1 Gew.-% enthalten.

Die Zubereitungen enthalten weiterhin gegebenenfalls Süßungsmittel, wie z. B. Saccharin, Aspartam oder Cyclamat. Kombinationen der Süßungsmittel kommen ebenfalls in Frage. Sie werden in den üblichen Konzentrationen eingesetzt, die in der Regel im Bereich von 0,05 bis 0,5 Gew.-% liegen.

Die Zubereitungen enthalten weiterhin gegebenenfalls Geschmacks- oder Aromastoffe. Bevorzugt sind Fleischaromen, wie z. B. "Artificial Beef Flavor" (Pharma Chemie, Inc., 1877 Midland Street, Syracuse, Nebr. 48666, insbesondere das Produkt mit der Bezeichnung PC-0125) sowie vorzugsweise Trockenleberpulver (z. B. vom Schwein oder vom Huhn). Die Geschmacks- oder Aromastoffe werden in den üblichen Konzentrationen eingesetzt, und zwar in der Regel im Bereich 1 bis 20 Gew.-%,. bevorzugt 2 bis 10 Gew.-%, besonders bevorzugt 3 bis 8 Gew.-%.

Die erfindungsgemäßen Zubereitungen können in an sich bekannter Weise hergestellt werden, indem man die Inhaltsstoffe in der Ölbasis löst bzw. dispergiert. Dabei kann es gegebenenfalls vorteilhaft sein, das Gemisch zu erwärmen. Falls eine feste Fettgrundlage eingesetzt wird, kann man z. B. so vorgehen, dass man die Ölbasis auf eine Temperatur erwärmt, bei der die feste Fettgrundlage schmilzt. Dann werden die Fettgrundlage und die anderen Inhaltstoffe unter Rühren zugegeben und man lässt die Mischung unter weiterem Rühren abkühlen.

Die erfindungsgemäßen Erzeugnisse eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so dass durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen. Als Beispiele seien genannt:
Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Anhyra spp, Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichlomosoides spp., Trichinella spp..

Aus der Ordnung des Rhabditia z.B.: Micronema spp., Strongyloides spp..

Aus der Ordnung der Strongylida z.B.: Strongylus spp., Triodontophorus spp., Oesophagodontus spp., Trichonema spp., Gyalocephalus spp., Cylindropharynx spp., Poteriostromum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostomum spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp., Cylicocyclus spp., Cylicodontophorus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Gruppe der Gigantohynchida z.B.: Filicollis spp., Moniliformis spp., Macracanthorhynchus spp., Prosthenorchis spp..

Maßgeblich ist dabei natürlich das Wirkungsspektrum des oder der eingesetzten Wirkstoffe. So wirken z. B. die Depsipeptide in der Regel gut gegen Trematoden, Nematoden, Acanthocephalen zeigen aber üblicherweise keine praktisch relevante Wirkung gegen Cestoden (Bandwürmer). Umgekehrt wirkt z. B. Praziquantel im wesentlichen nur gegen Cestoden.

Ebenfalls bevorzugt ist die Bekämpfung von Nematoden, wie z. B. aus der Ordnung der Strongylida z. B.: Ancylostoma spp., Uncinaria spp.,; Angiostrongylus spp.; Aerulostrongylus spp.; aus der Ordnung der Ascaridia z.B.: Toxocara spp.; Toxascaris spp.; aus der Ordnung der Filariida z.B.: Dirofilaria spp.

Besonders bevorzugt ist die Verwendung der erfindungsgemäßen Erzeugnisse zur Bekämpfung von Strongylida, insbesondere Ancylostoma spp., Uncinaria spp., sowie zur Bekämpfung von Ascaridia, insbesondere Toxocara spp., Toxascaris spp..

Sofern die Zubereitungen einen gegen Bandwürmer aktiven Wirkstoff enthalten, ist die Bekämpfung von z. B. Taenia spp. bevorzugt.

Parasitische Protozoen sind insbesondere Coccidien. Zu den Coccidien zählen:
Mastigophora (Flagellata) wie z. B. Trypanosomatidae z.B. Trypanosoma brucei, T.. gambiense, T. rhodesiense, T. congolense, T. cruzi, T. evansi, T. equinum, T. lewisi, T. percae, T. simiae, T. vivax, Leishmania brasiliensis, L. donovani, L. tropica, wie z.B. Trichomonadidae z.B. Giardia lamblia, G. canis, Sarcomastigophora (Rhizopoda) wie Entamoebidae z.B. Entamoeba histolytica, Hartmanellidae z. B. Acanthamoeba sp., Hartmanella sp., Apicomplexa (Sporozoa) wie Eimeridae z. B. Eimeria acervulina, E. adenoides, E. alabahmensis, E. anatis, E. anseris, E. arloingi, E. ashata, E. aubumensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuemii, Globidium spec., Isospora belli, I. canis, I. felis, I. ohioensis, I. rivolta, I. spec., I. suis, Neospora caninum, N. hugesi, Cystisospora spec., Cryptosporidium spec. wie Toxoplasmadidae z.B. Toxoplasma gondii, wie Sarcocystidae z.B. Sarcocystis bovicanis, S. bovihominis, S. neurona, S. ovicanis, S. ovifelis, S. spec., S. suihominis wie Leucozoidae z.B. Leucozytozoon simondi, wie Plasmodiidae z.B. Plasmodium berghei, P. falciparum, P. malariae, P. ovale, P. vivax, P. spec., wie Piroplasmea z.B. Babesia argentina, B. bovis, B. canis, B. spec., Theileria parva, Theileria spec., wie Adeleina z.B. Hepatozoon canis, H. spec..

Ferner Myxospora und Microspora z.B. Glugea spec. Nosema spec..

Ferner Pneumocystis carinii, sowie Ciliophora (Ciliata) wie z.B. Balantidium coli, Ichthiophthirius spec., Trichodina spec., Epistylis spec..

Ganz besonders hervorzuheben sind diejenigen Protozoen die zu den Apicomplexa gehören, vor allem Isospora spp., insbesondere Isospora canis und Isospora felis.

Zu den Nutztieren gehören insbesondere Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär. Von den Nutztier-Säugetieren bevorzugt sind Rinder, Schafe, und Schweine. Ebenfalls eingeschlossen für die erfindungsgemäße Verwendung sind die folgenden Tierarten, die keine Säugetiere sind, die aber ebenfalls zu den Nutztieren gehören: Vögel, wie z.B. Hühner, Gänse, Puten, Enten; Süß- und Salzwasserfische wie z.B. Forellen, Karpfen, Aale, Reptilien; Insekten wie z.B. Honigbiene und Seidenraupe.

Zu den Hobbytieren gehören bevorzugt Katzen und insbesondere Hunde. Bei diesen werden bevorzugt die o. g. Parasiten bei Katzenwelpen und insbesondere Hundewelpen bekämpft. Die Hundewelpen sind in der Regel 1 bis 6 Monate, bevorzugt 2 bis 14 Wochen alt.

Die Anwendung kann prophylaktisch oder therapeutisch erfolgen.

Die Anwendung der Zubereitungen erfolgt vorzugsweise oral.

Die Zubereitungen enthalten die Wirkstoffe jeweils in Konzentrationen von 10 ppm bis 90 Gew.-%, bevorzugt 50 ppm bis 50 Gew.-%, besonders bevorzugt 100 ppm bis 20 Gew.-%, bevorzugt von 100 ppm bis 10 Gew.-%.

Vorzugsweise enthalten die erfindungsgemäßen Zubereitungen 0,01 bis 3 Gew.-%, bevorzugt 0,05 bis 1 Gew.-%, besonders bevorzugt 0,1 bis 0,2 Gew.-% Depsipeptid.

Vorzugsweise enthalten die erfindungsgemäßen Zubereitungen 0,1 bis 10 Gew.-%, bevorzugt 1 bis 6 Gew.-%, besonders bevorzugt 2 bis 4 Gew.-% Triazin.

Das Gewichtsverhältnis von Depsipeptid zu Triazin in den erfindungsgemäßen Erzeugnissen hängt von verschiedenen Faktoren ab, liegt aber in der Regel im Bereich 1:99 bis 50: 50, bevorzugt 1:99 bis 30:70.

Die Dosierung des Triazins kann je nach Tierspezies variieren. Übliche Dosierungen liegen bei 1 bis 60 mg Wirkstoff pro kg Körpergewicht (mg/kg) des zu behandelnden Tieres pro Tag, bevorzugt 5 bis 40 mg/kg und besonders bevorzugt 10 bis 30 mg/kg.

Toltrazuril wird bei der oralen Anwendung normalerweise wie folgt dosiert (Dosierung pro Tag):

| | |
|---|---|
| Schwein: | 15 bis 25 mg/kg Körpergewicht, insbesondere etwa 20 mg/kg Körpergewicht |
| Rind: | 10 bis 20 mg/kg Körpergewicht, insbesondere etwa 15 mg/kg Körpergewicht |
| Schaf: | 15 bis 25 mg/kg Körpergewicht, insbesondere etwa 20 mg/kg Körpergewicht |
| Geflügel: | 10 bis 20 mg/kg Körpergewicht, insbesondere etwa 15 mg/kg Körpergewicht |
| Hund | 10 bis 20 mg/kg Körpergewicht, insbesondere etwa 15 mg/kg Körpergewicht |
| Katze | 10 bis 20 mg/kg Körpergewicht, insbesondere etwa 15 mg/kg Körpergewicht |

Außer bei Geflügel wird Toltrazuril pro Behandlung nur einmal verabreicht, sodass z.B. bei Schwein, Rind und Schaf die angegebenen Dosierungen sowohl pro Tag als auch pro Behandlung gelten. Bei Geflügel wird die angegebene Dosis auf zwei aufeinanderfolgende Tage verteilt.

Übliche Dosierungen der Depsipeptide liegen pro Tag bei 0,1 bis 100 mg/kg, bevorzugt 1 bis 50 mg/kg Körpergewicht pro Tag.

Beispielsweise wird Emodepside bei der oralen Anwendung üblicherweise wie folgt dosiert (Dosierung pro Tag):

| | |
|---|---|
| Hund: | 1 bis 5 mg/kg Körpergewicht |
| Hundewelpen: | 0,25 bis 2,5 mg/kg Körpergewicht |
| Rind: | 0,5 bis 5 mg/kg Körpergewicht |
| Schaf: | 0,5 bis 5 mg/kg Körpergewicht |

### Beispiele

Zur Herstellung der Beispiele werden die weiteren Inhaltsstoffe in der Ölbasis dispergiert. Falls einer festen Fettgrundlage wird die Ölbasis auf eine Temperatur erwärmt, bei der die feste Fettgrundlage schmilzt. Dann werden die Fettgrundlage und die anderen Inhaltstoffe unter Rühren zugegeben und man lässt die Mischung unter weiterem Rühren abkühlen.

In den Beispielen wird das Toltrazuril in mikronisierter Form eingesetzt und zwar mit d(90) < 15 µm.

| Beispiel 1 | |
|---|---|
| | % w/w |
| Toltrazuril | 2,0 |
| Emodepside | 0,1 |
| Butylhydroxyanisol | 0,1 |
| Kolloidales Siliziumdioxid (Aerosil 200) | 3,0 |
| Sorbinsäure | 0,08 |
| Sonnenblumenöl | ad 100,0 |
| | |

| Beispiel 2 | |
|---|---|
| | % w/w |
| Toltrazuril | 4,0 |
| Emodepsid | 0,1 |
| Butylhydroxyanisol | 0,1 |
| Kolloidales Siliziumdioxid (Aerosil 200) | 3,0 |
| Sorbinsäure | 0,08 |
| Sonnenblumenöl | ad 100,0 |
| | |

| Beispiel 3 | |
|---|---|
| | % w/w |
| Toltrazuril | 2,0 |
| Emodepsid | 0,1 |
| Butylhydroxyanisol | 0,1 |
| Glyceryldibehenat (Compritol 888ATO) | 3,5 |
| Sorbinsäure | 0,08 |
| Sonnenblumenöl | ad 100,0 |

| Beispiel 4 | |
|---|---|
| | % w/w |
| Toltrazuril | 4,0 |
| Emodepsid | 0,1 |
| Butylhydroxyanisol | 0,1 |
| Glyceryldibehenat (Compritol 888ATO) | 3,5 |
| Sorbinsäure | 0,08 |
| Sonnenblumenöl | ad 100,0 |
| | |

| Beispiel 5 | |
|---|---|
| | % w/w |
| Toltrazuril | 2,0 |
| Emodepsid | 0,1 |
| Butylhydroxyanisol | 0,1 |
| Glyceryldibehenat (Compritol 888ATO) | 5,0 |
| Sorbinsäure | 0,08 |
| Sonnenblumenöl | ad 100,0 |
| | |
| Beispiel 6 | |
| | % w/w |
| Toltrazuril | 2,0 |
| Emodepsid | 0,1 |
| Butylhydroxyanisol | 0,1 |
| Glyceryldibehenat (Compritol 888ATO) | 7,5 |
| Sorbinsäure | 0,08 |
| Sonnenblumenöl | ad 100,0 |
| | |

| Beispiel 7 | |
|---|---|
| | % w/w |
| Toltrazuril | 2,0 |
| Emodepsid | 0,1 |
| Butylhydroxyanisol | 0,1 |
| Glycerylpalmitostearat (Precirol ATO 5) | 3,5 |
| Sorbinsäure | 0,08 |
| Sonnenblumenöl | ad 100,0 |

| Beispiel 8 | |
|---|---|
| | % w/w |
| Toltrazuril | 2,0 |
| Emodepsid | 0,1 |
| Butylhydroxyanisol | 0,1 |
| Glycerylfettsäureester (Gelucire 43/01) | 3,5 |
| Sorbinsäure | 0,08 |
| Sonnenblumenöl | ad 100,0 |
| | |

| Beispiel 9 | |
|---|---|
| | % w/w |
| Toltrazuril | 2,0 |
| Emodepsid | 0,1 |
| Butylhydroxyanisol | 0,1 |
| Glyceryldibehenat (Compritol 888ATO) | 3,5 |
| Sorbinsäure | 0,08 |
| Miglyol 812 | ad 100,0 |
| | |

| Beispiel 10 | |
|---|---|
| | % w/w |
| Toltrazuril | 2,0 |
| Emodepsid | 0,1 |
| Butylhydroxytoluol | 0,1 |
| Glyceryldibehenat (Compritol 888ATO) | 3,5 |
| Sorbinsäure | 0,08 |
| Sonnenblumenöl | ad 100,0 |
| | |

| Beispiel 11 | |
|---|---|
| | % w/w |
| Toltrazuril | 2,0 |
| Emodepsid | 0,1 |
| Butylhydroxyanisol | 0,1 |
| Glyceryldibehenat (Compritol 888ATO) | 3,5 |
| Sorbinsäure | 0,08 |
| Sojaöl | ad 100,0 |
| | |

| Beispiel 12 | |
|---|---|
| | % w/w |
| Toltrazuril | 2,0 |
| Emodepsid | 0,1 |
| Butylhydroxytoluol | 0,1 |
| Glyceryldibehenat (Compritol 888ATO) | 3,5 |
| Sorbinsäure | 0,08 |
| Rhizinusöl | ad 100,0 |
| | |

| Beispiel 13 | |
|---|---|
| | % w/w |
| Toltrazuril | 2,0 |
| Emodepsid | 0,1 |
| Butylhydroxytoluol | 0,1 |
| Glyceryldibehenat (Compritol 888ATO) | 3,5 |
| Sorbinsäure | 0,08 |
| Sesamöl | ad 100,0 |
| | |

| Beispiel 14 | |
|---|---|
| | % w/w |
| Toltrazuril | 2,0 |
| Emodepsid | 0,1 |
| Butylhydroxytoluol | 0,1 |
| Glyceryldibehenat (Compritol 888ATO) | 3,5 |
| Sorbinsäure | 0,08 |
| Fischöl | ad 100,0 |
| | |

| Beispiel 15 | |
|---|---|
| | % w/w |
| Toltrazuril | 2,0 |
| Emodepsid | 0,1 |
| Butylhydroxyanisol | 0,04 |
| Glyceryldibehenat (Compritol 888ATO) | 2,1 |
| Sonnenblumenöl | ad 100,0 |
| | |

| Beispiel 16 | |
|---|---|
| | % w/w |
| Toltrazuril | 2,0 |
| Emodepsid | 0,1 |
| Butylhydroxyanisol | 0,2 |
| Sonnenblumenöl | ad 100,0 |
| | |
| Beispiel 17 | |
| | % w/w |
| Toltrazuril | 4,0 |
| Emodepsid | 0,1 |
| Butylhydroxyanisol | 0,2 |
| Aerosil 200 | 3,2 |
| Sorbinsäure | 0,5 |
| Sonnenblumenöl | ad 100,0 |
| | |

| Beispiel 18 | |
|---|---|
| | % w/w |
| Toltrazuril | 2,0 |
| Emodepsid | 0,05 |
| Butylhydroxyanisol | 0,2 |
| Glyceryldibehenat (Compritol 888ATO) | 3,5 |
| Sorbinsäure | 0,3 |
| Capryl-Caprinsäure-Triglycerid (Miglyol 812) | ad 100,0 |
| | |

| Beispiel 19 | |
|---|---|
| | % w/w |
| Toltrazuril | 2,0 |
| Emodepsid | 0,1 |
| Butylhydroxyanisol | 0,2 |
| Glyceryldibehenat (Compritol 888ATO) | 1,6 |
| Sonnenblumenöl | ad 100,0 |
| | |

| Beispiel 20 | |
|---|---|
| | % w/w |
| Toltrazuril | 2,0 |
| Emodepsid | 0,1 |
| Butylhydroxyanisol | 0,5 |
| Glyceryldibehenat (Compritol 888ATO) | 3,7 |
| Sorbinsäure | 0,5 |
| Sonnenblumenöl | ad 100,0 |
| | |

| Beispiel 21 | |
|---|---|
| | % w/w |
| Toltrazuril | 4,0 |
| Emodepsid | 0,1 |
| Butylhydroxyanisol | 0,5 |
| Glyceryldibehenat (Compritol 888ATO) | 3,7 |
| Sorbinsäure | 0,5 |
| Sonnenblumenöl | ad 100,0 |
| | |

| Beispiel 22 | |
|---|---|
| | % w/w |
| Toltrazuril | 2,0 |
| Emodepsid | 0,1 |
| Praziquantel | 1,0 |
| Butylhydroxyanisol | 0,1 |
| Glyceryldibehenat (Compritol 888ATO) | 5,0 |
| Sorbinsäure | 0,08 |
| Sonnenblumenöl | ad 100,0 |

### Biologische Beispiele

### A. Nematodenwirkung bei Hundewelpen

Zur Bestimmung der biologischen Wirksamkeit der Suspension gegen Nematoden wurden Hundewelpen artifiziell mit infektiösen Stadien unterschiedlicher Nematoden (s. Tabelle 1) infiziert. Nach Ermittlung einer patenten Infektion wurden die Tiere mit der Suspension in unterschiedlichen Dosierungen behandelt. Anhand von Kotuntersuchungen wurde der Rückgang der Eiausscheidung ermittelt, sowie die Anzahl der ausgeschiedenen Würmer. Fünf Tage nach Behandlung erfolgte eine zweite Behandlung mit einem Referenzprodukt. Danach wurden weiterhin Kotuntersuchungen durchgeführt und die Wirksamkeiten rechnerisch ermittelt.

**Tabelle 1**

| Anzahl Tiere | Nematoden | Dosierung Emodepside [mg/kg KG] | Wirksamkeit {%] | Formulierung |
|---|---|---|---|---|
| 6 pro Gruppe | *Uncinaria stenocephala* | 0.25 | 99.6 | Bsp. 15 Emodepsid 0.1% Toltrazuril 2 % |
| | | 0.1 | 85.7 | |
| | | 0.05 | 67.3 | |
| 6 pro Gruppe | *Uncinaria stenocephala* | 0.75 | 100 | Bsp. 16 Emodepsid 0.1% Toltrazuril 2 % |
| | | 0.5 | 100 | |
| | | 0.25 | 100 | |
| 6 pro Gruppe | *Toxocara canis* | 0.05 | 50.5 | Bsp. 17 Emodepsid 0.1% Toltrazuril 4 % |
| | | 0.125 | 98.7 | |
| | | 0.25 | 100 | |
| 8 pro Gruppe | *Ancylostoma caninum* | 0.25 | 94 | Bsp. 18 Emodepsid 0.05% Toltrazuril 2 % |
| | | 0.5 | 100 | |
| 13 | *Ancylostoma caninum* | 0.5 | 100 | Bsp. 19 Emodepsid 0.1% Toltrazuril 2 % |

### B. Wirkung gegen Isospora-Arten bei Hundewelpen

Zur Bestimmung der Wirksamkeit der Suspension gegen Isospora-Arten in Hunden wurden Welpen mit infektiösen Oocysten infiziert. Nach Ermittlung einer patenten Infektion anhand von Kotuntersuchungen wurden die Welpen mit unterschiedlichen Dosierungen behandelt und der therapeutische Behandlungserfolg gegen eine unbehandelte Kontrollgruppe ermittelt. Auch bei natürlich infizierten Tieren wurde die therapeutische Wirksamkeit der Suspension ermittelt. Weiterhin wurde der metaphylaktische Behandlungserfolg ermittelt, wobei Hundewelpen 3 bis 6 Tage nach Infektion behandelt wurden bevor eine patente Oocystenausscheidung ermittelt werden konnte. Der Behandlungserfolg wurde anhand von Kotuntersuchungen gegen eine unbehandelte Kontrolle ermittelt.

**Tabelle 2**

| Anzahl Tiere | Isosporaart | Behandlungs-Schema | Dosierung Toltrazuril [mg/kg KG] | Wirksamkeit {%] | Formulierung |
|---|---|---|---|---|---|
| 6 bis7 pro Gruppe | *Isospora ohioensis comp.* | metaphylaktisch | 10 | >99 | Bsp. 20 Emodepsid 0.1% Toltrazuril 2 % |
| | | therapeutisch | 10 | >99 | |
| | | therapeutisch | 20 | >99 | Bsp. 21 Emodepsid 0.1% Toltrazuril 4 % |
| 7 | *Isospora canis* | therapeutisch | 10 | >99 | Bsp. 20 Emodepsid 0.1% Toltrazuril 2 % |
| 9 pro Gruppe | *Isospora canis* | metaphylaktisch | 10 | 99.8 | Bsp. 10 Emodepsid 0.1% Toltrazuril 2 % |
| | | therapeutisch | 10 | 96.9 | |

## Patentansprüche

1. Zubereitungen enthaltend ein gegen parasitische Protozoen wirksames Triazin mit einer Komgröße d(90) ≤ 15 µm und ein anthelmintisches Cyclodepsipeptid in einer Ölbasis.

2. Zubereitung gemäß Anpruch 1, worin das Triazin eine Verbindung der Formeln (I) oder (II) ist 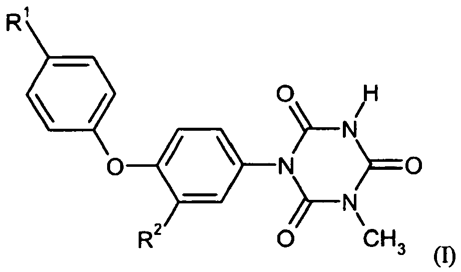 oder 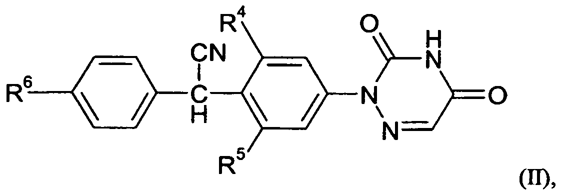 worin
R¹ für R³-SO₂- oder R³-S- steht,
R² für Alkyl, Alkoxy, Halogen oder SO₂N(CH₃)₂ steht und
R³ für Halogenalkyl steht
R⁴ und R⁵ unabhängig voneinander für Wasserstoff oder Cl stehen und
R⁶ für Fluor oder Chlor steht.
sowie ihre physiologisch verträglichen Salze

3. Zubereitung gemäß einem der vorstehenden Ansprüche, worin das Triazin Toltrazuril, Ponazuril oder Diclazuril ist.

4. Zubereitung gemäß einem der vorstehenden Ansprüche, worin das cyclische Depsipeptid ein 24-gliedriges Octacyclodepsipeptid ist.

5. Zubereitung gemäß Anspruch 4, worin das cyclische Depsipeptid Emodepsid ist.

6. Zubereitung gemäß Anspruch 4, worin das cyclische Depsipeptid PF 1022 ist.

7. Zubereitung gemäß einem der vorstehenden Ansprüche enthaltend ein Verdickungsmittel, insbesondere einen Glycerolester.

8. Zubereitung gemäß Anspruch 7 enthaltend als Verdickungsmittel einen Glyceroldiester mit C 12-C24-Fettsäuren.

9. Zubereitung gemäß Anspruch 8 enthaltend als Verdickungsmittel Glyceroldibehenat.

10. Zubereitung gemäß Anspruch 9 enthaltend:
| | |
|---|---|
| 2 - 4 Gew.-% | Toltrazuril |
| 0,1 - 0,2 Gew.-% | Emodepsid |
| 2 - 5 Gew.-% | Glyceryldibehenat |
| 0,05 - 0,5 Gew.-% | Butylhydroxytoluol |
| 0,02 - 0,1 Gew.-% | Sorbinsäure |
| ad 100 Gew.-% | Sonnenblumenöl |

11. Verwendung von Zubereitungen gemäß einem der vorstehenden Ansprüche zur Herstellung von Arzneimitteln zur Bekämpfung parasitären Protozoen und Endoparasiten bei Menschen oder Tieren.

12. Zubereitung gemäß einem der vorstehenden Ansprüche zur Bekämpfung von parasitären Protozoen und Endoparasiten bei Tieren.

## Claims

1. Preparations comprising a triazine which is active against parasitic protozoans and has a particle size d(90) ≤ 15 µm and an anthelmintic cyclodepsipeptide in an oil base.

2. Preparation according to Claim 1, where the triazine is a compound of the formulae (I) or (II) 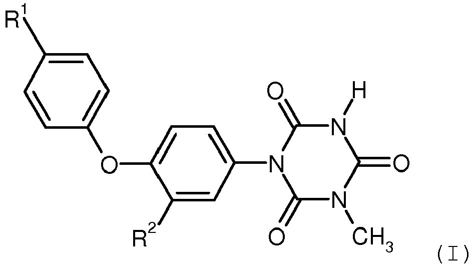 or 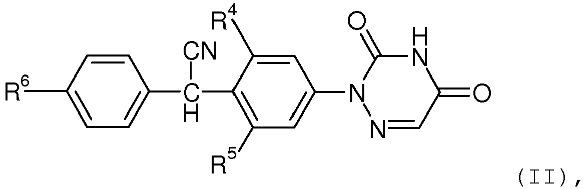 in which
R¹ represents R³-SO₂- or R³-S-,
R² represents alkyl, alkoxy, halogen or SO₂N(CH₃)₂ and
R³ represents haloalkyl,
R⁴ and R⁵ independently of one another represent hydrogen or Cl and
R⁶ represents fluorine or chlorine,
and their physiologically acceptable salts.

3. Preparation according to one of the preceding claims, where the triazine is toltrazuril, ponazuril or diclazuril.

4. Preparation according to one of the preceding claims, where the cyclic depsipeptide is a 24-membered octacyclodepsipeptide.

5. Preparation according to Claim 4, where the cyclic depsipeptide is emodepside.

6. Preparation according to Claim 4, where the cyclic depsipeptide is PF 1022.

7. Preparation according to one of the preceding claims, comprising a thickener, in particular a glycerol ester.

8. Preparation according to Claim 7, comprising, as thickener, a glycerol diester with C12-C24 fatty acids.

9. Preparation according to Claim 8, comprising, as thickener, glycerol dibehenate.

10. Preparation according to Claim 9, comprising:
2 - 4% by weight toltrazuril
0.1 - 0.2% by weight emodepside
2 - 5% by weight glyceryl dibehenate
0.05 - 0.5% by weight butylhydroxytoluene
0.02 - 0.1% by weight sorbic acid
to 100% by weight sunflower oil

11. Use of preparations according to one of the preceding claims for the preparation of pharmaceuticals for controlling parasitic protozoans and endoparasites in humans or animals.

12. Preparation according to one of the preceding claims for controlling parasitic protozoans and endoparasites in animals.

## Revendications

1. Préparations contenant une triazine efficace contre les protozoaires parasitiques ayant une taille de particule d(90) ≤ 15 µm et un cyclodepsipeptide anthelminthique dans une base huileuse.

2. Préparation selon la revendication 1, dans laquelle la triazine est un composé de formule (I) ou (II) 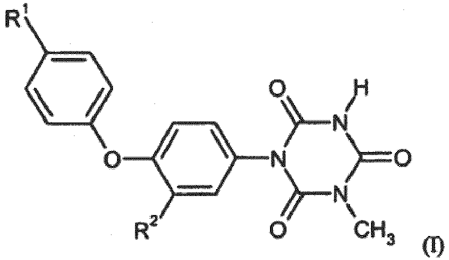 ou 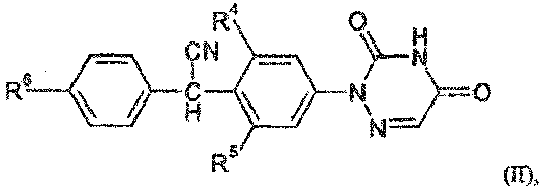 dans lesquelles
R¹ représente R¹-SO₂- ou R³-S-,
R² représente alkyle, alcoxy, halogène ou SO₂N(CH₃)₂ et
R³ représente halogénoalkyle
R⁴ et R⁵ représentent indépendamment l'un de l'autre hydrogène ou Cl, et
R⁶ représente fluor ou chlore,
ou ses sels physiologiquement compatibles.

3. Préparation selon l'une quelconque des revendications précédentes, dans laquelle la triazine est le toltrazuril, le ponazuril ou le diclazuril.

4. Préparation selon l'une quelconque des revendications précédentes, dans laquelle le depsipeptide cyclique est un octacyclodepsipeptide à 24 éléments.

5. Préparation selon la revendication 4, dans laquelle le depsipeptide cyclique est l'émodepside.

6. Préparation selon la revendication 4, dans laquelle le depsipeptide cyclique est PF 1022.

7. Préparation selon l'une quelconque des revendications précédentes, contenant un épaississant, notamment un ester de glycérol.

8. Préparation selon la revendication 7, contenant en tant qu'épaississant un diester de glycérol avec des acides gras en C12 à C24.

9. Préparation selon la revendication 8, contenant en tant qu'épaississant du dibéhénate de glycérol.

10. Préparation selon la revendication 9, contenant :
| | |
|---|---|
| 2 à 4 % en poids | de toltrazuril |
| 0,1 à 0,2 % en poids | d'émodepside |
| 2 à 5 % en poids | de dibéhénate de glycéryle |
| 0,05 à 0,5 % en poids | de butylhydroxytoluène |
| 0,02 à 0,1 % en poids | d'acide sorbique |
| jusqu'à 100 % en poids | d'huile de tournesol. |

11. Utilisation de préparations selon l'une quelconque des revendications précédentes pour la fabrication de médicaments pour lutter contre les protozoaires parasitiques et les endoparasites chez les hommes ou les animaux.

12. Préparation selon l'une quelconque des revendications précédentes pour lutter contre les protozoaires parasitiques et les endoparasites chez les animaux.
